(19) Europäisches Patentamt
European Patent Office
Office européen des brevets

(11) **EP 2 104 855 B1**

(12) **FASCICULE DE BREVET EUROPEEN**

(45) Date de publication et mention de la délivrance du brevet:
**31.10.2012 Bulletin 2012/44**

(51) Int Cl.:
**G01N 33/46** *(2006.01)*   **G01N 29/07** *(2006.01)*
**G01N 3/40** *(2006.01)*   **G01N 27/04** *(2006.01)*

(21) Numéro de dépôt: **06847203.4**

(22) Date de dépôt: **21.12.2006**

(86) Numéro de dépôt international:
**PCT/FR2006/051413**

(87) Numéro de publication internationale:
**WO 2008/074929 (26.06.2008 Gazette 2008/26)**

(54) **PROCÉDÉ ET DISPOSITIF POUR DÉTERMINER LA QUALITÉ, CLASSER ET TRIER UN MATÉRIAU HYGROSCOPIQUE**

VERFAHREN UND VORRICHTUNG ZUR BESTIMMUNG DER QUALITÄT EINES HYGROSKOPISCHEN MATERIALS UND ZUR KLASSIFIZIERUNG UND SORTIERUNG DAVON

METHOD AND DEVICE FOR DETERMINING THE QUALITY OF A HYGROSCOPIC MATERIAL AND FOR CLASSIFYING AND SORTING IT

(84) Etats contractants désignés:
**AT BE BG CH CY CZ DE DK EE ES FI FR GB GR HU IE IS IT LI LT LU LV MC NL PL PT RO SE SI SK TR**

(43) Date de publication de la demande:
**30.09.2009 Bulletin 2009/40**

(73) Titulaire: **Tecsan SARL**
**1950 Sion 2 (CH)**

(72) Inventeur: **SANDOZ, Jean-Luc**
**1110 Morges (VD) (CH)**

(74) Mandataire: **Palix, Stéphane et al**
**Cabinet Laurent & Charras**
**"Le Contemporain"**
**50, Chemin de la Bruyère**
**69574 Dardilly Cedex (FR)**

(56) Documents cités:
**EP-A1- 0 634 655   EP-B1- 0 456 776**
**WO-A-2005/078435   US-A1- 2005 086 023**

• **SANDOZ J L ET AL: "Wood poles ageing and non destructive testing tool" ELECTRICITY DISTRIBUTION. PART 1: CONTRIBUTIONS. CIRED. 14TH INTERNATIONAL CONFERENCE AND EXHIBITION ON (IEE CONF. PUBL. NO. 438), LONDON, UK,IEE, UK, vol. 3, 2 juin 1997 (1997-06-02), pages 26-1, XP006506393 ISBN: 0-85296-674-1**

**Description**

La présente invention concerne un procédé pour déterminer la qualité d'un matériau hygroscopique. Le procédé selon la présente invention permet également de classer dans une catégorie le matériau hygroscopique, en fonction de la détermination de sa qualité. Le procédé conformément à cette invention permet de trier le matériau hygroscopique, en fonction de son classement. L'invention se rapporte en outre à un dispositif destiné à mettre en oeuvre ce procédé.

Dans le domaine de la construction en bois, et plus spécialement dans le domaine des structures en bois, les nouvelles normes européennes en vigueur donnent des classes de bois de structure, qui sont définies par rapport à leurs performances mécaniques. Pour les bois massifs, la norme européenne EN 338 définit par exemple neuf classes de bois de structure pour les essences résineuses ($C_{14}$, $C_{16}$, $C_{18}$, $C_{22}$, $C_{24}$, $C_{27}$, $C_{30}$, $C_{35}$ et $C_{40}$) et trois classes pour les bois feuillus ($D_{50}$, $D_{60}$ et $D_{70}$). L'indice de la classe correspond à la valeur garantie de résistance à la rupture en flexion.

Cette norme EN 338 demande que pour chaque planche classée, quelque soit l'essence de bois considérée, les trois valeurs techniques suivantes soient données:

- la résistante en flexion, exprimée en $N/mm^2$ ;
- le module d'élasticité, permettant de calculer les déformations, exprimé en $N/mm^2$ ; et
- la densité, ou masse volumique, exprimée en $kg/m^3$, pour calculer la performance des assemblages.

La norme européenne EN 14081 précise les tests de qualification à faire pour homologuer une machine capable de trier les bois de structure selon la norme EN 338. Enfin, pour le bois lamellé collé, c'est la norme européenne EN 1194 qui définit quatre classes ($GL_{24}$, $GL_{28}$, $GL_{32}$ et $GL_{36}$).

Ces divers matériaux de construction, en particulier le bois, sont hygroscopiques, c'est-à-dire qu'une de leur propriété est d'absorber l'humidité de l'air. Les propriétés mécaniques de ces matériaux sont de ce fait difficiles à déterminer, en raison de leur variation en fonction du degré ou taux d'humidité.

Art antérieur

Les principales technologies de classement des bois, fonctionnant en ligne automatique dans des scieries (fabricants des composants planches) ou dans les entreprises de charpente (production de produits en bois collé ou de systèmes constructifs utilisant des bois massifs à la base), sont regroupées dans les familles suivantes.

On distingue ainsi le « stress grader », dans lequel la planche est déformée en flexion avec une force constante. Si la déformation est trop importante, la planche est déclassée (en classes inférieures $C_{14}$, $C_{16}$, $C_{18}$ et $C_{22}$). Si la déformation est faible ou très faible, la planche est surclassée ($C_{30}$ et $C_{40}$). Cette méthode donne une performance locale, mesurée sur toute la longueur de la planche. Cependant, un réglage spécifique pour chaque section testée est nécessaire.

Dans la mesure dynamique, une extrémité d'une planche, posée sur ses supports, est excitée. La fréquence de vibration de la planche donne son module d'élasticité dynamique, donc sa classe de performance. Cette méthode donne une valeur globale de performance. Cependant, la machine doit être étalonnée par section, par longueur, et par nombre de supports.

Les méthodes de mesure par rayonnement, type rayons X ou rayons $\gamma$, déterminent la masse volumique du bois. Cette propriété est déduite en analysant la fonction d'absorption du rayonnement. Ces méthodes travaillent transversalement et mesurent de faibles épaisseurs, inférieure à 75 mm. Cependant, cette méthode ne donne que la masse volumique, ce qui est insuffisant pour un classement mécanique. Cette machine doit donc être combinée à une autre technologie.

Certaines machines combinent plusieurs des méthodes précédentes pour améliorer la qualité de l'évaluation non destructive de la performance mécanique, et la mesure simultanée du module d'élasticité, de la résistance en flexion et de la masse volumique.

Cependant, ces machines s'avèrent particulièrement complexes à installer, à étalonner et à piloter, faisant augmenter rapidement les coûts. Les machines combinant plusieurs mesures sont plus difficiles à étalonner, parce que le modèle le plus exact possible doit être trouvé sur la base de plusieurs données non destructives. Ensuite, en augmentant le nombre de mesures physiques, par exemple les noeuds ou la densité par rayons X, le rendement de la machine en pièces de haute performance diminue. En effet, en mesurant des défauts comme le noeud, la pièce peut être déclassée alors que sa qualité est bonne. De plus, avec plusieurs paramètres, la limite de la machine est vite atteinte. Par exemple, avec une mesure de densité par rayons X, l'épaisseur maximale mesurable est de 75 mm, même si les autres variables sont encore mesurables.

Le document EP- 0.456.776 décrit un appareil pour la détection automatique de la classe de propriétés mécaniques d'une pièce de bois. Cet appareil fonctionne par le principe de la mesure du temps de propagation d'un signal ultrasonique à l'intérieur d'une telle pièce. Plus précisément, cet appareil est composé d'une sonde émettrice, connectée à un générateur d'un signal à ultrason, mise en place sur une des faces de la pièce de bois, et d'une sonde réceptrice disposée sur la face opposée à celle qui reçoit la sonde émettrice.

La mesure du décalage temporel entre l'émission du signal ultrasonique par la sonde d'émission et la réception par la sonde réceptrice permet de déterminer le temps de propagation de l'onde ultrasonique à l'intérieur de la pièce de bois. En fonction de la valeur du temps de propagation, rapporté à la longueur sur laquelle l'onde se propage, on détermine la vitesse de propagation, et en conséquence l'appartenance de la pièce à telle catégorie de qualité. Cet appareil comprend également des capteurs d'humidité et de température permettant de corriger la valeur de vitesse ainsi calculée pour la comparer avec des mesures de vitesse normalisées en température et humidité.

Bien que donnant grande satisfaction, cet appareil ne permet pas de détecter certains changements de qualité à l'intérieur de la pièce de bois, dont l'influence est neutre sur la vitesse de propagation des ondes ultrasoniques. Par exemple, la présence de noeuds, de fissures ou de poches de dégradation biologique ne produit pas automatiquement une variation de la vitesse de propagation suffisamment sensible pour être identifiée à coup sûr. Pour certaines essences, des défauts caractéristiques peuvent rendre une pièce inutilisable ou sans valeur, alors que la zone atteinte ne produit aucun changement de vitesse de propagation des ondes ultrasoniques. En outre, une mesure d'autres paramètres physiques n'est pas réalisée.

On connaît, d'après le document EP- 0.634.655 un dispositif permettant de contrôler, de manière simple, efficace et rapide l'état physique d'un support en bois, tel qu'un poteau pour lignes électriques ou téléphoniques. Ce dispositif permet de mesurer simultanément la dureté et l'hygroscopie de ce support en bois. Ce dispositif portatif est particulièrement bien adapté et permet de contrôler aisément de nombreux supports en bois directement sur leur site d'installation.

Cependant, ce dispositif ne tient pas compte d'autres paramètres importants qui peuvent être, par exemple, la nature, l'âge et le séchage du bois, pouvant apporter des modifications et des variations dans les mesures de dureté et d'hygroscopie. En outre, avec uniquement deux variables mesurées, la dureté et l'hygroscopie, l'interprétation des valeurs s'avère complexe et peut même conduire à des résultats erronés. Ce dispositif n'est donc pas suffisant pour permettre de trier et de choisir des matériaux hygroscopiques, n'ayant pas encore servis, de différentes sections, par exemple rectangulaire, et aptes à être utilisés dans le domaine de la construction.

Le document US- 4.838.085 décrit un procédé et un dispositif pour réaliser un test non destructif et une évaluation des propriétés d'un panneau réalisé avec différents types de matériaux composites. Le procédé permet de déterminer les défauts, notamment de collage. Le matériau composite est soumis à un signal physique, sous la forme d'ultrasons se propageant dans le panneau. Au moins deux sondes de détection permettent de mesurer la vitesse de propagation des ultrasons. Des moyens d'analyse et de calcul sont prévus pour déterminer les propriétés mécaniques en fonction de la valeur de densité estimée, de la vitesse et du taux d'atténuation des ultrasons dans le matériau.

Cependant, dans un tel procédé, la valeur de densité est estimée dès le départ pour une série de panneaux. Il y a donc une simple indication d'ordre de grandeur de densité, sans mesure spécifique. Aucune mesure du taux d'humidité n'est faite, ce qui rend ce procédé très peu approprié pour des matériaux hygroscopiques tels que du bois.

Exposé de l'invention

Un problème principal que se propose de résoudre l'invention consiste à mettre au point un procédé simple et rapide de détermination des propriétés mécaniques de différents matériaux hygroscopiques. Un deuxième problème est de concevoir un procédé qui permette d'intégrer de nouveaux paramètres propres au matériau de construction, afin d'accroître la fiabilité des résultats de diagnostic obtenus. Un troisième problème est la mise en oeuvre d'un procédé de détermination de l'état et de la qualité d'un matériau, à partir de plusieurs paramètres mesurables. Un quatrième problème est de prévoir un procédé et un dispositif correspondant permettant de comparer, de classer et de trier des matériaux hygroscopiques.

Dans la présente invention, un procédé sert à déterminer la qualité d'un matériau hygroscopique, tel que du bois ou autres matériaux de construction analogues.

Conformément à un premier aspect de l'invention, le procédé tel que défini par la revendication 1 comprend les étapes consistant :

- à mesurer la vitesse de propagation d'ondes sonores (V) à l'intérieur du matériau hygroscopique,
- à mesurer le taux d'humidité (H) du matériau hygroscopique,
- à mesurer la dureté (F) du matériau hygroscopique, et
- à calculer des valeurs de masse volumique (p), de module d'élasticité (E) et de résistance à la rupture en flexion (R) pour le matériau hygroscopique, en fonction de la vitesse de propagation d'ondes sonores mesurée (V), du taux d'humidité mesuré (H) et de la dureté mesurée (F).

Autrement dit, à partir de la mesure de trois paramètres, qui sont vitesse de propagation d'ondes sonores, taux d'humidité et dureté, les valeurs de masse volumique, de module d'élasticité et de résistance à la rupture en flexion sont calculées, donnant une idée précise de la qualité du matériau. Si la pièce est bonne par mesure aux ultrasons, combinée avec la dureté, le paramètre noeud est négligé. Ceci permet d'augmenter les rendements de mesure et ainsi les performances du dispositif de mise en oeuvre du procédé.

De préférence, le procédé peut en outre comprendre les étapes consistant :

- à comparer les valeurs calculées de la masse volumique (p), du module d'élasticité (E) et de la résistance à la rupture en flexion (R), par rapport à une table de référence comprenant un ensemble de valeurs préenregistrées de masse volumique, de module d'élasticité et de résistance à la rupture en flexion, pour un matériau hygroscopique de même type de référence,
- à établir un classement du matériau hygroscopique en fonction de l'étape de comparaison, et
- à réaliser un triage du matériau hygroscopique en fonction de l'étape de classement.

L'étape consistant à calculer la masse volumique (p) du matériau hygroscopique peut se faire conformément à l'équation p = $\alpha$.F + $\delta$, dans laquelle (F) est la dureté mesurée du matériau hygroscopique et ($\alpha$) et ($\delta$) sont des coefficients d'étalonnage.

Le procédé peut en outre comprendre, de manière particulièrement favorable, une étape consistant à corriger les valeurs de vitesse de propagation d'ondes sonores mesurées (V) et de masse volumique calculée (p), de façon à les rapporter à un taux d'humidité de 12 % et à une température de 20°C.

L'étape consistant à calculer le module d'élasticité (E) du matériau hygroscopique peut avantageusement se faire conformément à l'équation E = $V^2.\rho$, dans laquelle (V) est la vitesse de propagation d'ondes sonores mesurée et (p) est la masse volumique calculée pour le matériau hygroscopique.

L'étape consistant à calculer la résistance à la rupture en flexion (R) du matériau hygroscopique peut se faire préférentiellement conformément à l'équation R = $\beta V + \gamma \rho + \chi E + \varepsilon$, dans laquelle (V) est la vitesse de propagation, (p) est la masse volumique, (E) est le module d'élasticité et ($\beta$), ($\gamma$), ($\chi$) et ($\varepsilon$) étant des coefficients d'étalonnage.

Le procédé peut en outre comprendre une étape consistant à mesurer la masse du matériau hygroscopique. La valeur de masse va permettre de corriger la valeur de masse volumique calculée (p), afin d'apporter plus de précisions, par exemple pour les bois s'avérant difficiles à trier.

Conformément à un deuxième aspect de la présente invention, un dispositif tel que défini par la revendication 8 de détermination de la qualité d'un matériau hygroscopique est destiné à mettre en oeuvre le procédé tels que décrit ci-dessus.

Le dispositif peut comprendre des moyens de mesure de la vitesse de propagation d'ondes sonores (V) à l'intérieur du matériau hygroscopique, selon un axe longitudinal perpendiculaire à une surface frontale du matériau. Le dispositif peut comprendre des moyens de mesure de la dureté (F) et du taux d'humidité (H) du matériau hygroscopique selon un axe transversal perpendiculaire à une surface supérieure du matériau. Le dispositif peut comprendre des moyens de mesure de la masse du matériau hygroscopique. Le dispositif comprend des moyens de calcul des valeurs de masse volumique ($\rho$), de module d'élasticité (E) et de résistance à la rupture en flexion (R) pour le matériau hygroscopique, en fonction de la vitesse de propagation d'ondes sonores mesurée (V), du taux d'humidité mesuré (H) et de la dureté mesurée (F).

Les moyens de calcul peuvent être couplés à des moyens de triage du matériau hygroscopique, en fonction de la comparaison des valeurs de masse volumique ($\rho$), de module d'élasticité (E) et de résistance à la rupture en flexion (R) avec des valeurs de référence de masse volumique ($\rho$), de module d'élasticité (E) et de résistance à la rupture en flexion (R).

Description sommaire des figures

L'invention sera bien comprise et ses divers avantages et différentes caractéristiques ressortiront mieux lors de la description suivante, de l'exemple non limitatif de réalisation, en référence aux dessins schématiques annexés, dans lesquels :

- la Figure 1 représente une vue en perspective d'un dispositif de détermination de la qualité, de classement et de triage de matériaux hygroscopiques ;
- la Figure 2 représente une vue latérale d'un détail du dispositif de la Figure 1 ;

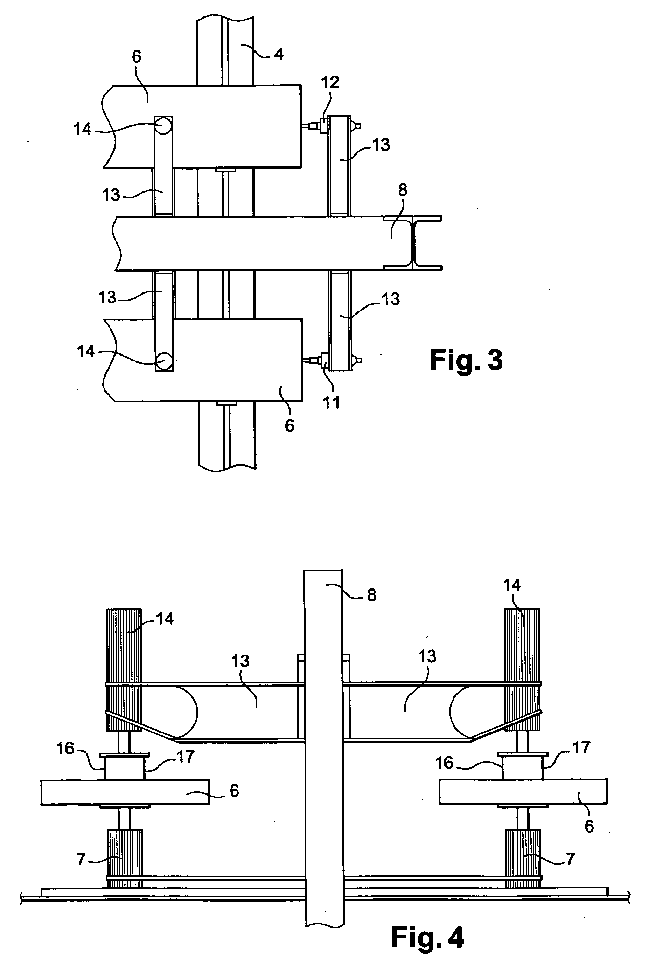

- la Figure 3 représente une vue du dessus d'un détail du dispositif de la Figure 1 ; et
- la Figure 4 représente une vue frontale d'un détail du dispositif de la Figure 1.

Description détaillée de l'invention

Selon le procédé de l'invention, à partir d'une mesure des paramètres physiques non destructifs sur un élément de bois, c'est-à-dire acousto-ultrasoniques de vitesse (V), dureté (F) et taux d'humidité (H), il est calculé les valeurs de masse volumique ($\rho$), de module d'élasticité (E) et de résistance à la rupture en flexion (R).

La mesure acousto-ultrasonique est basée sur l'analyse de propagation d'une onde basse fréquence dans le bois. La mesure acousto-ultrasonique est donc basée sur la mesure de deux paramètres non destructifs, combinant deux phénomènes physiques pour être plus efficace.

La première information obtenue par cette mesure acousto-ultrasonique est le temps de propagation de l'onde donnant la vitesse de propagation dans la planche.

En mode longitudinal, cette vitesse ($V_L$) est corrélée au module d'élasticité (E), donc à la performance mécanique. Elle intègre partiellement les défauts comme les noeuds, qui en détournant la fibre, augmentent les temps de propagation. Par exemple pour de l'épicéa, les planches de hautes performances mécaniques atteignent des vitesses de 5500 m/s à 6500 m/s pour des ondes d'excitation d'environ 20 kHz.

En mode transversal, cette vitesse ($V_R$) est dépendante de la masse volumique ($\rho$) du bois, dans le cas d'un panneau de particules par exemple, ou de la qualité d'un collage, pour un produit stratifié.

La deuxième information obtenue par cette mesure acousto-ultrasonique est la réponse acoustique de la planche en énergie (atténuation), qui est significative des défauts internes de la planche, tels que les noeuds ou les zones mal collées qui sont des ressorts mous constituant des points faibles. Les défauts absorbent beaucoup d'énergie, et l'énergie transmise après propagation est donc plus faible sur une planche noueuse ou sur une lame de parquet mal collée. La résistance de la planche est très sensible à ces points faibles qui généreront la rupture.

Fondamentalement, la mesure de la vitesse de propagation de l'onde ultrason basse fréquence ($V_L$ et $V_R$) traduit le coefficient de performance ($C_{LL}$ et $C_{RR}$) du bois, sur ses deux axes longitudinaux (L) et transversaux (R) par rapport aux fibres, selon les équations suivante :

$$V_L{}^2 = C_{LL}/\rho \approx E_L/\rho \quad \text{et} \quad V_R{}^2 = C_{RR}/\rho \approx E_R/\rho$$

La vitesse ($V_L$ et $V_R$) donne directement la performance du bois, c'est-à-dire le rapport de rigidité (élasticité $E_L$ et $E_R$) par unité de masse volumique. On peut ainsi admettre une relation approchée entre la vitesse (V) et le module d'élasticité (E).

La variabilité naturelle du bois donne pour une essence, par exemple pour l'épicéa, une variation :

- de module d'élasticité (E), de 6000 N/mm$^2$ à 24000 N/mm$^2$, soit un facteur de quatre entre les plus faibles et les plus fortes ; et
- de masse volumique ($\rho$), de 360 kg/m$^3$ à 490 kg/m$^2$, soit une variation inférieure à 1,5 entre les plus légères et les plus lourdes.

De ce fait, si la masse volumique ($\rho$) est connue en détail planche par planche et non plus estimée en valeur moyenne donnée pour une essence de bois, la relation entre vitesse (V) et module d'élasticité (E) devient bien supérieure, puisque l'équation permet de calculer directement le module d'élasticité (E). La masse volumique ($\rho$) est calculée à partir de la mesure de la dureté (F). La dureté (F) est mesurée avec un dispositif sensiblement analogue à celui décrit dans le document EP- 0.634.655.

Dans une autre réalisation, la valeur de masse volumique calculée ($\rho$) est corrigée par une connaissance de la masse de la planche. Une étape de pesée intervient et peut s'avérer utile pour compléter la mesure de dureté, qui donne seulement une information très locale et indirectement corrélée à la densité. Connaître la masse de la planche s'avère utile, pour améliorer la corrélation finale sur les performances, notamment pour des bois difficiles à trier.

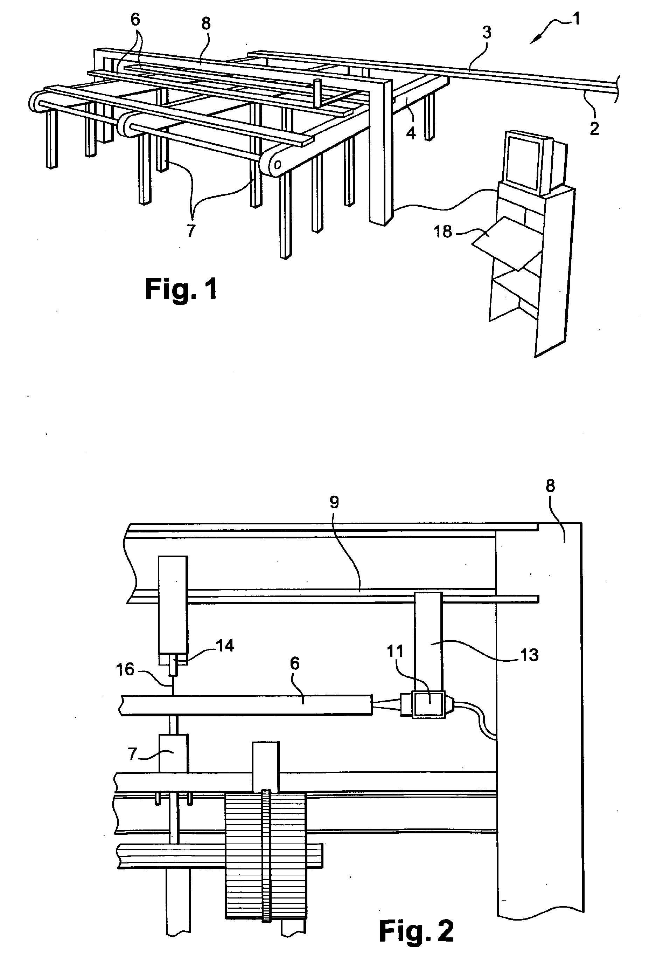

Comme le montre la Figure 1, un dispositif sous la forme d'une machine automatique (1) met en oeuvre ce procédé. La machine (1) réalise et reproduit de manière consécutive pour chaque planche amenée une par une les phases décrites ci-après. L'échantillon à tester peut être une planche en bois, un panneau de particules, un panneau contre-plaqué aggloméré, un panneau bois massif collé (parquet), un panneau stratifié, un panneau bois-ciment, une poutre, un tronc, une grume ou billon de bois avant sciage, des matériaux à base de fibres ou d'autres équivalents.

Des planches sont successivement transférées par une chaîne de transport ou de convoyage au niveau des têtes de mesure acousto-ultrasonique (AUS). Une chaîne de transport (2) amène les planches (3) sur un châssis de pré-positionnement (4) avant le test, puis les évacue après le test. La chaîne de transport (2) et le châssis de pré-positionnement (4) sont équipés de taquets (non visibles en détail) qui positionnent une planche devant être testée (6) dans une géométrie fixe par rapport à une sonde ultrason, donc pour obtenir un couplage géométriquement constant dans le plan XY.

Pour que les paramètres AUS soient mesurés précisément, il est nécessaire de développer des moyens capables de reproduire très précisément les mêmes conditions de mesures avec des ondes introduites de façon constante dans la planche à tester (6) et avec un couplage des deux transducteurs, l'émetteur et le récepteur, très reproductible, surtout au niveau de la pression de couplage, donc de la surface d'émission et de réception.

Pour ce faire, la planche à tester (6) est soulevée par un châssis de mesure intermédiaire (7), complètement désolidarisé des convoyeurs de la chaîne de transport (2). Le châssis de mesure (7) supporte la planche à tester (6) pour la phase des tirs du test ultrason, de façon à éviter les pollutions vibratoires, pour éliminer toute excitation par vibration parasitaire de la planche à tester (6), donc pour permettre une mesure AUS précise et reproductible. Ce châssis de mesure (7) présente des appuis élastomères pour les contacts de support de la planche à tester (6), permettant d'atténuer rapidement son excitation due au déplacement dans la machine (1).

Un pont de mesure (8) supporte l'axe de déplacement (9) de deux sondes ultrasons (11 et 12), qui sont maintenues par un bridage métallique (13). Les sondes (11 et 12) sont les mêmes en émission et en réception. Les deux sondes émettrices et réceptrices (11 et 12) sont couplées avec la même pression de couplage, par exemple sensiblement égale à 20 kg pour chaque planche à tester (6). Les sondes (11 et 12) peuvent être numérisées pour connaître leur position géométrique sur l'axe de mesure et déduire ainsi la longueur de la planche à tester (6), permettant alors un calcul de la vitesse de propagation (V) en fonction de la mesure du temps de propagation.

Les deux sondes (11 et 12) réalisent un déplacement axial, pour une mesure parallèle aux fibres. Les deux sondes (11 et 12) réalisent un déplacement transversal par rapport à la planche à tester (6), pour une mesure transversale. Les deux sondes (11 et 12) peuvent également se placer en position indirecte à 30° pour des planches très longues. La machine (1), de par son système dé positionnement de la planche à tester (6), permet une reproduction du point de couplage constant, par rapport aux extrémités de la planche à tester (6).

La mesure AUS est réalisée pour recevoir les deux paramètres, temps de propagation et énergie transmise. Après la mesure, la paire de sondes (11 et 12) est découplée de la planche à tester (6).

Une mesure de la masse de la planche (6) se fait par pesée à l'aide de pesons introduits sous les appuis de la planche (6), quand cette dernière est positionnée pour être mesurée par les ultrasons.

Après la mesure AUS, la planche à tester (6) est avancée au niveau du capteur (14) de masse volumique (p), où le test de dureté (F) est réalisé. Ce test se fait en enfonçant une paire d'aiguilles (16 et 17) dans la planche à tester (6), dans le sens perpendiculaire à la largeur de la planche à tester (6). La mesure se fait perpendiculaire aux fibres, donc perpendiculaire à la planche à tester (6).

La force de compression nécessaire à l'enfoncement de la paire d'aiguilles (16 et 17) dans le bois est mesurée. Le capteur (14) est réglable en hauteur, pour avoir une pénétration qui correspond à environ 80 % de l'épaisseur de la planche à tester (6), c'est-à-dire par exemple sur une épaisseur de pénétration de 20 mm à 50 mm, selon l'épaisseur de la planche de bois à mesurer. La technologie de la tête de mesure de la dureté (F) est réglable par l'opérateur pour une pénétration par palier de 20 mm, 30 mm, 40 mm et 50 mm, variant en fonction de l'épaisseur de la planche à tester (6).

Après que le capteur de force ait mesuré la dureté (F), les aiguilles (16 et 17) restent enfoncées dans le bois et une tension leur est ensuite appliquée. La mesure de l'hygroscopie (H) est ainsi réalisée sur la base du principe résistif. La mesure a lieu en tête des aiguilles (16 et 17), là où elles ne sont pas isolées (pointes extrêmes).

Les informations de métrologie, à savoir vitesse ou énergie (V), masse, dureté (F) et hygroscopie (H), sont transmises au logiciel qui calcule ensuite la qualité du bois. Le logiciel corrige les paramètres mesurés pour les ramener à une valeur de taux d'humidité égal à 12 % et à une température de 20°C. Le logiciel calcule la masse volumique (ρ), le module d'élasticité (E) et la résistance à la rupture en flexion (R), conformément aux équations présentées ci-dessus. Le cas échéant, le logiciel corrige la valeur de masse volumique (ρ), en fonction de la masse du bois. La qualité du bois est ensuite déterminée en fonction du résultat de ces calculs et par comparaison à une table de valeurs préenregistrées.

Une armoire (18) permet le rangement des cartes électroniques du système de mesure des paramètres temps et énergie de propagation. Une carte électronique permet la gestion multiplexe de plusieurs paires de sondes travaillant simultanément. Un ordinateur permet de faire travailler un logiciel qui fait les calculs, communique avec l'opérateur pour connaître les choix et les destinations. Ce logiciel transmet des ordres aux cartes électroniques relais qui activent la chaîne de transfert et des box de qualité (non représenté).

La planche venant d'être testée est ensuite évacuée dans le box de qualité correspondant aux qualités supérieures, qualités moyennes, qualités inférieures ou rejet. Le cycle est recommencé avec la planche à tester (6) suivante. La cadence de mesure atteint 4 secondes pour un cycle complet, c'est-à-dire 15 fois par minutes, ce qui donne le nombre de planches par test par minute.

Pour améliorer la qualité des mesures, la machine (1) est pilotée par un logiciel qui va fixer certains paramètres en fonction du type et des sections de bois (essence) et de la longueur à mesurer (de quelques mm à quelques mètres, par exemple 10 m), parmi lesquels :

- le niveau d'amplification du signal reçu sur la sonde réceptive AUS, pour mesurer le temps et l'énergie de façon précise ;

- le filtrage fréquentiel du signal AUS transmis pour ne traiter que la plage de fréquence constituant la plage de vraisemblance de la mesure des deux paramètres AUS et filtrer les pollutions acoustiques ;
- le nombre de tirs AUS supérieur à un, pour réaliser un calcul statistique du résultat (par exemple 10 tirs, avec analyse de la moyenne sur les quatre résultats les plus proches, donc les plus probables) ;
- la limite de sélectivité des deux paramètres AUS et de la dureté, donc la qualité des planches classées, en fonction de la norme EN 338 ;
- la destination des planches par qualité (box de stockage provisoire) pour évacuer en mode automatique, la bonne qualité dans le bon box ;
- le prépositionnement de la sonde réceptive sur la machine de façon à ce que cette sonde ne se déplace que de quelques cm pour réaliser le couplage, et permettre ainsi une cadence plus rapide ; et
- la pénétration à régler de la tête de mesure pour la dureté (F).

Par ailleurs, pour améliorer l'évaluation de la qualité, cette machine (1) a une interface avec l'opérateur et permet de gérer au niveau de ses algorithmes, par exemple par paramétrage des coefficients d'étalonnage $(\alpha)$ $(\beta)$, $(\gamma)$, $(\chi)$ et $(\varepsilon)$, différents paramètres d'influence sur les performances mécaniques, parmi lesquels :

- le type de bois (rond, scié, collé, panneaux, etc...) ;
- le type de planches (section, longueur) ;
- l'essence (sapin, épicéa, pin, chêne, bois exotiques ou autres) ;
- l'hygroscopie ou le taux d'humidité lié au bois ;
- la température de l'élément mesuré ;
- le temps de propagation de l'onde ;
- l'énergie acoustique transmise par le bois ;
- le nombre de têtes de mesure ; et
- le mode de propagation de l'onde (qui peut-être longitudinal, longitudinal indirect ou transversal) par rapport aux fibres du bois.

La présente invention n'est pas limitée aux modes de réalisation décrits et illustrés. De nombreuses modifications peuvent être réalisées, sans pour autant sortir du cadre défini par la portée du jeu de revendications.

**Revendications**

1.   Procédé pour déterminer la qualité d'un matériau hygroscopique, tel que du bois ou autres matériaux de construction analogues, comprenant les étapes consistant :

- à mesurer la vitesse de propagation d'ondes sonores (V) à l'intérieur du matériau hygroscopique,
- à mesurer le taux d'humidité (H) du matériau hygroscopique,
- à mesurer la dureté (F) du matériau hygroscopique,
- à calculer des valeurs de masse volumique $(\rho)$, de module d'élasticité (E) et de résistance à la rupture en flexion (R) pour le matériau hygroscopique, en fonction de la vitesse de propagation d'ondes sonores mesurée (V), du taux d'humidité mesuré (H) et de la dureté mesurée (F).

2.   Procédé selon la revendication 1, **caractérisé en ce qu'**il comprend en outre les étapes consistant :

- à comparer les valeurs calculées de la masse volumique $(\rho)$, du module d'élasticité (E) et de la résistance à la rupture en flexion (R), par rapport à une table de référence comprenant un ensemble de valeurs préenregistrées de masse volumique, de module d'élasticité et de résistance à la rupture en flexion, pour un matériau hygroscopique de même type de référence,
- à établir un classement du matériau hygroscopique en fonction de l'étape de comparaison, et
- à réaliser un triage du matériau hygroscopique en fonction de l'étape de classement.

3.   Procédé selon la revendication 1 ou 2, **caractérisé en ce que** l'étape consistant à calculer la masse volumique $(\rho)$ du matériau hygroscopique se fait conformément à l'équation :

$$\rho = \alpha.F + \delta$$

dans laquelle (F) est la dureté mesurée du matériau hygroscopique et ($\alpha$) et ($\delta$) sont des coefficients d'étalonnage.

4. Procédé selon l'une quelconque des revendications précédentes, **caractérisé en ce qu'**il comprend en outre une étape consistant à corriger les valeurs de vitesse de propagation d'ondes sonores mesurées (V) et de masse volumique calculée ($\rho$), de façon à les rapporter à un taux d'humidité de 12 % et à une température de 20°C.

5. Procédé selon l'une quelconque des revendications précédentes, **caractérisé en ce que** l'étape consistant à calculer le module d'élasticité (E) du matériau hygroscopique se fait conformément à l'équation :

$$E = V^2.\rho$$

dans laquelle (V) est la vitesse de propagation d'ondes sonores mesurée et ($\rho$) est la masse volumique calculée pour le matériau hygroscopique.

6. Procédé selon la revendication 5, **caractérisé en ce que** l'étape consistant à calculer la résistance à la rupture en flexion (R) du matériau hygroscopique se fait conformément à l'équation :

$$R = \beta V + \gamma\rho + \chi E + \varepsilon$$

dans laquelle (V) est la vitesse de propagation, ($\rho$) est la masse volumique, (E) est le module d'élasticité et ($\beta$), ($\gamma$), ($\chi$) et ($\varepsilon$) étant des coefficients d'étalonnage.

7. Procédé selon l'une quelconque des revendications précédentes, **caractérisé en ce qu'**il comprend en outre une étape consistant à mesurer la masse du matériau hygroscopique, de façon à corriger la valeur de masse volumique calculée ($\rho$).

8. Dispositif de détermination de la qualité d'un matériau hygroscopique (6), **caractérisé en ce qu'**il est destiné à mettre en oeuvre le procédé selon l'une quelconque des revendications précédentes, et **en ce qu'**il comprend :

   - des moyens de mesure de la vitesse de propagation d'ondes sonores (V) à l'intérieur du matériau hygroscopique,
   - des moyens de mesure du taux d'humidité (H) du matériau hygroscopique,
   - des moyens de mesure de la dureté (F) du matériau hygroscopique, et
   - des moyens de calcul (18) des valeurs de masse volumique ($\rho$), de module d'élasticité (E) et de résistance à la rupture en flexion (R) pour le matériau hygroscopique (6), en fonction de la vitesse de propagation d'ondes sonores mesurée (V), du taux d'humidité mesuré (H) et de la dureté mesurée (F).

9. Dispositif selon la revendication 8, **caractérisé en ce qu'**il comprend des moyens de mesure (11, 12) de la vitesse de propagation d'ondes sonores (V) à l'inférieur du matériau hygroscopique (6) selon un axe longitudinal perpendiculaire à une surface frontale du matériau (6).

10. Dispositif selon la revendication 8 ou 9, **caractérisé en ce qu'**il comprend des moyens de mesure (14, 16, 17) de la dureté (F) et du taux d'humidité (H) du matériau hygroscopique (6), selon un axe transversal perpendiculaire à une surface supérieure du matériau (6).

11. Dispositif selon l'une quelconque des revendications 8 à 10, **caractérisé en ce qu'**il comprend des moyens de mesure de la masse du matériau hygroscopique (6).

12. Dispositif selon la revendication 8, **caractérisé en ce que** les moyens de calcul sont couplés à des moyens de triage du matériau hygroscopique (6), en fonction de la comparaison des valeurs de masse volumique ($\rho$), de module

d'élasticité (E) et de résistance à la rupture en flexion (R) avec des valeurs de référence de masse volumique (ρ), de module d'élasticité (E) et de résistance à la rupture en flexion (R).

**Claims**

1. A method for determining the quality of a hygroscopic material, such as wood or other similar building materials, comprising the steps of:

    - measuring the propagation velocity of sound waves (V) within the hygroscopic material,
    - measuring themoisture content (H) of the hygroscopic material,
    - measuring the hardness (F) of the hygroscopic material,
    - calculating the values of density (p),elasticity modulus (E) and flexural tensile strength (R) for the hygroscopic material, as a function of the measured sound wave propagation velocity (V), measuredmoisture content (H) and measured hardness (F).

2. The method according to claim 1, **characterized in that** it further comprises the steps of:

    - comparing the calculated values of density (ρ), elasticity modulus (E) and flexuraltensile strength(R), with respect to a reference table comprising a set of pre-recorded valuesof density, elasticity modulus and flexuraltensile strength for a hygroscopic material having the same type of reference,
    - ranking the hygroscopic material according to the comparison step, and
    - sorting the hygroscopic material in accordance with the ranking step.

3. The method according to claim 1 or 2, **characterized in that** the step of calculating the density (ρ) of the hygroscopic material is performed according to the equation:

$$\rho = \alpha . F + \delta$$

wherein (F) is the measured hardness of the hygroscopic material and ($\alpha$) and ($\delta$) are calibration coefficients.

4. The method according to any one of the preceding claims, **characterized in that** it further comprises a step of correcting the values of the measured sound wave propagation velocity (V) and calculated density (ρ), so as to determine their ratio with respect to amoisture content of 12% and a temperature of 20°C.

5. The method according to any one of the preceding claims, **characterized in that** the step of calculating the elasticity modulus (E) of the hygroscopic material is performed according to the equation:

$$E = V^2 . \rho$$

wherein(V) is the measured sound wave propagation velocity and $\rho$ is the calculated density for the hygroscopic material.

6. The method according to claim 5, **characterized in that** the step of calculating the flexuraltensile strength (R) of the hygroscopic material is performed according to the equation:

$$R = \beta V + \gamma \rho + \chi E + \varepsilon$$

wherein (V) is the propagation velocity, (p) is the density, (E) is the elasticity modulus, with ($\beta$) , ($\gamma$), ($\chi$) and ($\varepsilon$) being calibration coefficients.

7. The method according to any one of the preceding claims, **characterized in that** it further comprises a step of measuring the mass of the hygroscopic material in order to correct the calculated density value (ρ).

8. A device for determining the quality of a hygroscopic material (6), **characterized in that** it is intended to implement the method according to any one of the preceding claims, and **in that** it comprises:

   - means for measuring the propagation velocity (V)of sound waves within the hygroscopic material,
   - means for measuring the moisture content of the hygroscopic material,
   - means for measuring the hardness (F) of the hygroscopic material, and
   - means for calculating(18) the values ofdensity ($\rho$), elasticity modulus (E) and flexural tensile strength(R) for the hygroscopic material (6), as a function of the measured sound wave propagation velocity (V), measured moisture content (H) and measured hardness (F).

9. The device according to claim 8, **characterized in that** it comprises means for measuring (11, 12) the propagation velocity of sound waves (V) within the hygroscopic material (6) along a longitudinal axis perpendicular to the front surface of the material (6).

10. The device according to claim 8 or 9, **characterized in that** it comprises means (14, 16, 17) for measuring the hardness (F) and moisture content(H) of the hygroscopic material (6), along atransverse axis perpendicular to an upper surface of the material (6).

11. The device according to any one of claims 8 to 10, **characterized in that** it comprises means for measuring the mass of the hygroscopic material (6).

12. The device according to claim 8, **characterized in that** the calculating means are coupled with means for sorting the hygroscopic material (6), accordingto the comparison between the values of density ($\rho$), elasticity modulus (E) and flexuraltensile strength (R) and the reference values of density ($\rho$), elasticity modulus (E) and flexuraltensile strength (R).


**Patentansprüche**

1. Verfahren zum Bestimmen der Qualität eines hygroskopischen Materials wie etwa von Holz oder anderen analogen Baumaterialien, die Schritte umfassend, die darin bestehen:

   - die Schallwellenausbreitungsgeschwindigkeit (V) im Inneren des hygroskopischen Materials zu messen,
   - den Feuchtigkeitsgehalt (H) des hygroskopischen Materials zu messen,
   - die Härte (F) des hygroskopischen Materials zu messen,
   - die Werte der Dichte ($\rho$), des Elastizitätsmoduls (E) und der Bruchdehnungsfestigkeit (R) für das hygroskopische Material in Abhängigkeit von der gemessenen Schallwellenausbreitungsgeschwindigkeit (V), dem gemessenen Feuchtigkeitsgehalt (H) und der gemessenen Härte (F) zu berechnen.

2. Verfahren nach Anspruch 1, **dadurch gekennzeichnet, dass** es darüber hinaus die Schritte umfasst, die darin bestehen:

   - die berechneten Werte der Dichte ($\rho$), des Elastizitätsmoduls (E) und der Bruchdehnungsfestigkeit (R) im Hinblick auf eine Bezugstabelle zu vergleichen, die eine Gesamtheit vorab gespeicherter Dichtewerte, Elastizitätsmodulwerte und Bruchdehnungsfestigkeitswerte für ein hygroskopisches Material derselben Bezugsart umfasst,
   - eine Klassierung des hygroskopischen Materials in Abhängigkeit vom Vergleichsschritt herzustellen, und
   - eine Sortierung des hygroskopischen Materials in Abhängigkeit vom Klassierungsschritt durchzuführen.

3. Verfahren nach Anspruch 1 oder 2, **dadurch gekennzeichnet, dass** der Schritt, der darin besteht, die Dichte (p) des hygroskopischen Materials zu berechnen, entsprechend der Gleichung:

$$\rho = \alpha \cdot F + \delta$$

erfolgt, worin (F) die gemessene Härte des hygroskopischen Materials ist und es sich bei ($\alpha$) und ($\delta$) um Kalibrierungskoeffizienten handelt.

**4.** Verfahren nach einem der vorhergehenden Ansprüche, **dadurch gekennzeichnet, dass** es darüber hinaus einen Schritt umfasst, der darin besteht, die gemessenen Werte der Schallwellenausbreitungsgeschwindigkeit (V) und der berechneten Dichte (ρ) zu korrigieren, um sie in Bezug auf einen Feuchtigkeitsgehalt von 12% und eine Temperatur von 20°C zu setzen.

**5.** Verfahren nach einem der vorhergehenden Ansprüche, **dadurch gekennzeichnet, dass** der Schritt, der darin besteht, den Elastizitätsmodul (E) des hygroskopischen Materials zu berechnen, entsprechend der Gleichung:

$$E = V^2 \bullet \rho$$

erfolgt, worin (V) die gemessene Schallwellenausbreitungsgeschwindigkeit und (ρ) die berechnete Dichte für das hygroskopische Material ist.

**6.** Verfahren nach Anspruch 5, **dadurch gekennzeichnet, dass** der Schritt, der darin besteht, die Bruchdehnungsfestigkeit (R) des hygroskopischen Materials zu berechnen, entsprechend der Gleichung:

$$R = \beta V + \gamma \rho + \chi E + \varepsilon$$

erfolgt, worin (V) die Ausbreitungsgeschwindigkeit, (ρ) die Dichte, (E) der Elastizitätsmodul ist, und es sich bei (β), (γ), (χ) und (ε) um Kalibrierungskoeffizienten handelt.

**7.** Verfahren nach einem der vorhergehenden Ansprüche, **dadurch gekennzeichnet, dass** es darüber hinaus einen Schritt umfasst, der darin besteht, die Masse des hygroskopischen Materials zu messen, um den berechneten Wert der Dichte (ρ) zu korrigieren.

**8.** Vorrichtung zum Bestimmen der Qualität eines hygroskopischen Materials (6), **dadurch gekennzeichnet, dass** sie dazu bestimmt ist, das Verfahren nach einem der vorhergehenden Ansprüche durchzuführen, und dass sie umfasst:

- Einrichtungen zum Messen der Schallwellenausbreitungsgeschwindigkeit (V) im Inneren des hygroskopischen Materials,
- Einrichtungen zum Messen des Feuchtigkeitsgehalts (H) des hygroskopischen Materials,
- Einrichtungen zum Messen der Härte (F) des hygroskopischen Materials, und
- Einrichtungen (18) zum Berechnen von Werten der Dichte (ρ), des Elastizitätsmoduls (E) und der Bruchdehnungsfestigkeit (R) für das hygroskopische Material (6) in Abhängigkeit von der gemessenen Schallwellenausbreitungsgeschwindigkeit (V), des gemessenen Feuchtigkeitsgehalts (H) und der gemessenen Härte (F).

**9.** Vorrichtung nach Anspruch 8, **dadurch gekennzeichnet, dass** sie Einrichtungen (11, 12) zum Messen der Schallwellenausbreitungsgeschwindigkeit (V) im Inneren des hygroskopischen Materials (6) entlang einer zu einer Frontfläche des Materials (6) senkrechten Achse umfasst.

**10.** Vorrichtung nach Anspruch 8 oder 9, **dadurch gekennzeichnet, dass** sie Einrichtungen (14, 16, 17) zum Messen der Härte (F) und des Feuchtigkeitsgehalts (H) des hygroskopischen Materials (6) entlang einer zu einer Oberfläche des Materials (6) quer verlaufenden Achse umfasst.

**11.** Vorrichtung nach einem der Ansprüche 8 bis 10, **dadurch gekennzeichnet, dass** sie Einrichtungen zum Messen der Masse des hygroskopischen Materials (6) umfasst.

**12.** Vorrichtung nach Anspruch 8, **dadurch gekennzeichnet, dass** die Einrichtungen zum Berechnen mit Einrichtungen zum Sortieren des hygroskopischen Materials (6) in Abhängigkeit vom Vergleich der Werte der Dichte (ρ), des Elastizitätsmoduls (E) und der Bruchdehnungsfestigkeit (R) mit Bezugswerten der Dichte (ρ), des Elastizitätsmoduls (E) und der Bruchdehnungsfestigkeit (R) verbunden sind.

EP 2 104 855 B1

**Fig. 1**

**Fig. 2**

Fig. 3

Fig. 4

EP 2 104 855 B1

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0456776 A
- EP 0634655 A

- US 4838085 A